# EUROPÄISCHE PATENTSCHRIFT

(11) **EP 2 446 812 B1**
(45) Veröffentlichungstag und Bekanntmachung des Hinweises auf die Patenterteilung: **28.12.2016**
(21) Anmeldenummer: 10405203.0
(22) Anmeldetag: 26.10.2010
(51) Int. Cl.: A61B 3/00, A61B 3/14, A61B 3/13, A61B 3/135

(54) **Augenuntersuchungsgerät mit digitaler Bildausgabe**
Device for examining eyes with digital imaging
Appareil d'analyse oculaire doté d'une sortie d'image numérique

(43) Veröffentlichungstag der Anmeldung: 02.05.2012
(73) Patentinhaber: HAAG-STREIT AG, 3098 Köniz (CH)
(72) Erfinder: Teijido, Juan Manuel, 3098 Schliern (CH)
(74) Vertreter: Rüfenacht, Philipp Michael

(56) Entgegenhaltungen:
- EP-A1- 1 908 399
- WO-A1-2006/000072
- WO-A1-2009/043494
- DE-A1- 3 737 935
- DE-A1- 19 541 477
- DE-A1- 19 720 851
- JP-A- 5 245 109
- US-A- 5 912 720

## Beschreibung

### Technisches Gebiet

Die Erfindung betrifft eine Vorrichtung zur Untersuchung eines Auges mit einer Optik zur Erzeugung eines Bildes des Auges.

### Stand der Technik

Spaltlampenmikroskope sind ophthalmologische Untersuchungsgeräte, mit welchen die Augen monoskopisch oder stereoskopisch untersucht werden können. Bekannte Spaltlampenmikroskope haben zur stereoskopischen Betrachtung eines Auges eine Optik zum Erzeugen zweier Bilder eines Auges und zwei Okulare zur stereoskopischen Betrachtung der Bilder sowie eine Beleuchtungseinheit. Die Beleuchtungseinheit ist auf einem vertikal verlaufenden Ast einer Halteeinheit angeordnet. Das zu betrachtende Auge ist in einer annähernd horizontal verlaufenden Ebene auf einer Seite der Halteeinheit positionierbar. Die Beleuchtungseinheit umfasst eine Glühlampe, LEDs oder ähnliche Beleuchtungsmittel für eine Spaltbeleuchtung.

Gegenwärtige Vorrichtungen zur Untersuchung und/oder Behandlung eines Auges umfassen eine Digitalkamera zum Aufnehmen eines Bildes sowie einen Bildschirm zur Wiedergabe des Bildes.

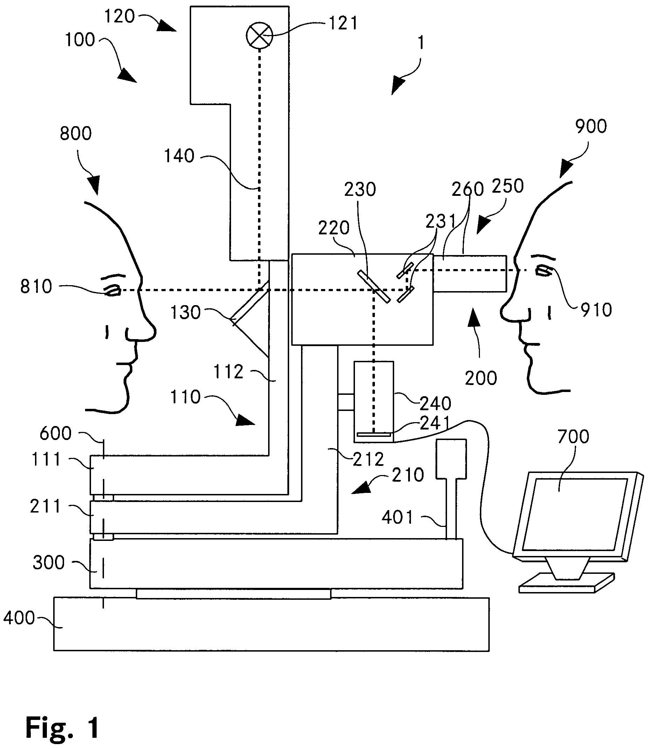

Vorrichtungen zur stereoskopischen Untersuchung eines Auges, bei welchen Bilddaten elektronisch erfasst werden, sind aus dem Stand der Technik bekannt:
Die US 5 912 720 A (Berger) betrifft ein computerbasiertes ophthalmologisches Untersuchungsgerät, mittels welchem aktuelle und ältere Bilddaten überlagert werden können und welches für Telemedizin und Unterricht geeignet ist. Dazu umfasst eines der beiden Okulare der Spaltlampe einen Strahlteiler und eine CCD-Kamera. Ein Teil des Bildes für ein Auge wird über den Strahlteiler zum Betrachter geleitet, während der andere Teil zur CCD-Kamera gelangt. Von der CCD-Kamera wird das Bildmaterial an ein Computersystem zur Verarbeitung, Darstellung an einem Bildschirm und zur Speicherung in einem Speicher übertragen. Das mit der CCD-Kamera aufgenommene Bildmaterial kann über einen Mikrobildschirm und einen Strahlteiler mit dem Echtzeit-Bildmaterial des Mikroskops für ein Auge des Betrachters überlagert werden. Als Erweiterung der Figur 1 kann die Überlagerung auch für beide Augen erfolgen, womit eine stereoskopische Darstellung erreicht werden kann.
Die DE 37 37 935 A1 (Proxitronic) betrifft ein Stereo-Farbfernseh-Mikroskop für die Augenmedizin. Der Augenhintergrund wird vom Licht einer Spaltlampe beleuchtet und durch eine Optik, über einen Strahlenteiler von je einer Schwarzweiss-Fernsehkamera in den beiden Stereokanälen des Mikroskops aufgenommen. Vor jeder Kamera ist jeweils ein Farbfilter, nämlich ein Grün und Rot- bzw. Blaufilter vorgesehen. Die beiden Signale für den linken und den rechten Stereokanal werden schliesslich über eine Codierschaltung dem Eingang je eines Farbfernesehmonitors zugeführt auf deren Bildschirm das linke und das rechte Farbbild dargestellt werden kann und über je ein Okular mit Strahlumlenkoptik vom Betrachter beobachtet werden können.
Die WO 2006/000072 A1 (Mitre) betrifft eine Vorrichtung und ein Verfahren zum Bereitstellen, Speichern und Wiedergeben von stereoskopischen Bildern in der Ophthalmologie. Die Vorrichtung umfasst ein stereoskopisches Mikroskop, welches zwei optische Systeme für das linke und das rechte Auge aufweist. Zwischen den Objektivlinsen und den entsprechenden Okularen sind jeweils ein Strahlteiler vorgesehen, wobei jeweils ein erster Teilstrahl zu den Okularen und der zweite Teilstrahl jeweils zu den Kameras geleitet werden um die Bilder in elektrische Signale umzuwandeln. Die Daten können in einem Speicher aufgezeichnet werden oder in Echtzeit auf einem oder mehreren Bildschirmen wiedergegeben und mittels einer Shutterbrille betrachtet werden. Alternativ können die Kameras auch direkt mit den entsprechenden Okularen verbunden sein, wobei der Rest der Vorrichtung gleich bleibt.
Die DE 197 20 851 A1 (Carl Zeiss) betrifft ein Spaltlampenmikroskop, bei welchem zwischen einem Objektiv und Tubuslinse für jedes Betrachterauge ein separater, paralleler Strahlengang besteht und wobei eine Auskopplung des Beobachtungsstrahlengangs in Richtung einer Videokamera erfolgen kann. Eine stereoskopische Betrachtung wird dadurch ermöglicht, dass für die jeweiligen Zwischenbilder anstelle der Okulare jeweils Videokameras vorgesehen sind. Die Bilddaten werden in das Auge des Betrachters, unter Verwendung einer VRD-Brille, eingespiegelt.
Die WO 2009/043494 A1 (Carl Zeiss) betrifft ein ophthalmologisches Gerät zur Erzeugung von Bildern. Die Vorrichtung umfasst mindestens zwei Bildsensoren, weiche in denselben Strahlengang eingespiegelt sind, so dass mehrere Bilder mit identischem Szeneninhalt und identischer Bewegungsschärfe mit erweiterter Dynamik (unterschiedlicher Belichtungszeit) aufgenommen werden können.
Die DE 195 41 477 A1 (Nidek) betrifft Spaltlampenmikroskope zur konfokalen abtastmikroskopischen Beobachtung. Die Abtastmikroskopeinheit enthält eine Lichtquelle, eine Kondensorlinse, Filter, eine Blende, einen Polarisator und einen Strahlteiler. Weiter umfasst sie eine Nipkow Scheibe mit spiralförmig angeordneten Löchern, welche mit hoher Geschwindigkeit drehbar ist. Das durch den Spiegel umgelenkte Licht wird über Spiegel in zwei Lichtstrahlen geteilt, so dass der Untersucher die Beobachtungsebene des Auges als Stereobild betrachten kann.

Diese Vorrichtungen haben den Nachteil, dass die Koordination bei der Bedienung der Vorrichtung und der gleichzeitigen Betrachtung des Bildmaterials auf dem Bildschirm für den Untersuchenden ungewohnt ist. Die Betrachtung über einen Bildschirm während der Untersuchung ist weiter nicht optimal, da der Bildschirm eine gewisse Distanz vom Betrachter aufweisen muss, damit der Betrachter einfach und schnell auf den Bildschirm fokussieren kann. Die Positionierung ist in beinahe jedem Fall für den Untersuchenden und den Untersuchten sowie aus konstruktiven Gründen ungünstig.

### Darstellung der Erfindung

Aufgabe der Erfindung ist es, eine dem eingangs genannten technischen Gebiet zugehörende Vorrichtung zur stereoskopischen Untersuchung eines Auges zu schaffen, welche eine verbesserte Ergonomie und Bedienung ermöglicht.

Die Lösung der Aufgabe ist durch die Merkmale des Anspruchs 1 definiert. Gemäss einem ersten Aspekt der Erfindung umfasst die Vorrichtung wenigstens einen, vorzugsweise zwei Bildsensoren zur elektronischen Erfassung der beiden Bilder.

Damit wird erreicht, dass das Bildmaterial, insbesondere zur stereoskopischen Betrachtung, aufbereitet werden kann. Der Bildsensor kann jeweils ein sogenanntes Halbbild erfassen, womit eine dreidimensionale Darstellung erreicht werden kann. Typischerweise repräsentiert ein Halbbild jeweils die Sicht eines Auges auf einen Gegenstand, das heisst, die beiden Halbbilder stellen im Wesentlichen denselben Gegenstand unter zwei verschiedenen Betrachtungswinkeln dar. Damit kann das Bildmaterial nachträglich, oder auch im Wesentlichen in Echtzeit, elektronisch aufbereitet werden. Die Aufbereitung kann dabei verschiedenste, insbesondere bekannte Bild- und Filmbearbeitungstechniken umfassen. Als Beispiel seien hier Kontrast, Helligkeit, Farben, Hilfslinien (Gitternetze etc.), Abstrahierung des Bildmaterials, Bildüberlagerung (zum Vergleichen zweier Bilder oder eben zur stereoskopischen Darstellung derselben) etc. erwähnt.

Die Vorrichtung zur stereoskopischen Untersuchung eines Auges (nachfolgend Vorrichtung genannt), insbesondere das Spaltlampenmikroskop kann weitgehend konventionell, das heisst in bekannter Art und Weise aufgebaut sein.

Die Optik umfasst gemäss einem ersten Aspekt der Erfindung zwei Objektive, welche unter unterschiedlichen Winkeln denselben Punkt fokussieren können, so dass aus den beiden Bilddaten ein stereoskopisches Bild erhalten werden kann. Das Linsensystem der Objektive kann speziell auf den respektive die Bildsensoren abgestimmt sein, womit sie sich unter Umständen von konventionellen Objektiven einer Spaltlampe unterschieden können. Dem Fachmann ist klar, wie die entsprechende Optik zur Erzeugung zweier Bilder des Auges zur anschliessenden stereoskopischen Betrachtung ausgebildet sein kann.

Der Bildsensor kann genügend gross ausgebildet sein, so dass auf diesem die zwei Bilder nebeneinander zeitgleich aufgenommen werden können. Der Bildsensor kann die beiden Halbbilder auch zeitlich alternierend aufnehmen, insbesondere wenn der Sensor in genügend hoher Frequenz, zum Beispiel grösser oder gleich 100 Hz, Einzelbilder verarbeiten kann. Ein Nachteil kann sich allerdings dadurch ergeben, dass mit demselben Bildsensor nur die halbe Bildfrequenz erreicht werden kann. Bei schnellen Augenbewegungen des betrachteten Auges können dadurch die Bildpaare zur stereoskopischen Betrachtung nicht genügend aufeinander abgestimmt respektive zu wenig synchron sein, so dass der 3D-Effekt bei der Betrachtung eine zu geringe Qualität aufweisen kann. Je nach Höhe der Aufnahmefrequenz des Bildsensors kann dies aber auch in Kauf genommen werden, insbesondere da damit die Vorrichtung kompakter ausgebildet werden kann. In einer weiteren bevorzugten Variante ist eine Anwendung einer Interlace-Technik vorgesehen, bei welcher die Pixelsensoren zeilenweise alternierend das linke respektive das rechte Halbbild aufnehmen. Die Pixelreihen können dazu alternierend so ausgebildet sein, dass zum Beispiel mittels geeigneter Linsen ein Lichtstrahl von einer ersten Pixelreihe nur dann erfasst wird, wenn dieser einen bestimmten Einfallwinkel α aufweist, während eine zweite Pixelreihe einen Lichtstrahl nur dann erfasst, wenn dieser einen Einfallwinkel β # α aufweist, womit die beiden Halbbilder auseinander gehalten werden können.

In einer weiteren bevorzugten Ausführungsform umfasst die Vorrichtung für das linke und das rechte Bild jeweils einen eigenen Bildsensor oder Bildsensorbereich, so dass die maximal mögliche Bildfrequenz des Bildsensors ausgeschöpft werden kann.

Die Vorrichtung gemäss dem ersten Aspekt der Erfindung kann so ausgebildet sein, dass ein Bild durch den Sensor erfasst und gleichzeitig mittels eines herkömmlichen Okulars als analoges Bild betrachtet werden kann. Dazu kann in der Vorrichtung zum Beispiel ein Strahlteiler vorgesehen sein, wobei ein Teilstrahl vom zu untersuchenden Auge zum Okular und ein weiterer Teilstrahl zum Sensor geleitet werden kann. Die Optik zur Erzeugung der Bilder kann, insbesondere falls keine analoge Betrachtungsmöglichkeit vorgesehen ist, auf den Bildsensor abgestimmt respektive optimiert sein.

Der Bildsensor respektive der optoelektronische Sensor kann als CCD-Sensor (Chargecoupled Device), CMOS-Sensor (Complementary Metal Oxide Semiconductor), DPS-Sensor (digital Pixel Sensor), aber auch als Bildsensor für andere Spektralbereiche ausgebildet sein. Dem Fachmann sind dazu weitere geeignete Vorrichtungen zum elektronischen Erfassen von Bildmaterial respektive Filmmaterial, bekannt. Schliesslich können auch mehr als zwei Bildsensoren vorgesehen sein. Zum Beispiel können zwei verschiedene Bildsensortypen für unterschiedliche Spektralbereiche vorgesehen sein, so dass gesamthaft vier Bildsensoren vorhanden sind.

Vorzugsweise umfasst die Vorrichtung eine Betrachtungseinheit mit zwei Bildwiedergabeeinheiten zur Darstellung der beiden Bilder sowie zwei Okularen zur stereoskopischen Betrachtung der Bilder. Bevorzugt ist die Betrachtungseinheit über eine Datenleitung mit dem Bildsensor so verbunden, dass die durch den Bildsensor elektronisch aufgenommenen Bilder in Form von elektronischen Daten an die Betrachtungseinheit übermittelt werden können. Die Bilddaten werden innerhalb der Betrachtungseinheit bevorzugt an zwei Bildwiedergabeeinheiten gesandt. Die Bildwiedergabeeinheiten empfangen die Bilddaten und wandeln diese in ein für einen Betrachter sichtbares Bild um. Die Bildwiedergabeeinheiten sind dazu vorzugsweise als elektronische Bildschirme, insbesondere als zwei Mikrobildschirme ausgebildet. Die Bildschirme können als bekannte t-LCD- (transmissive Liquid Crystal Display), LCoS (Liquid Crystal on Silicon), OLED-(organic light emitting diode) oder als optisches MEMS (Micro Electromechanical System) ausgebildet sein. Die Bildwiedergabeeinheiten werden bevorzugt durch jeweils ein Okular, insbesondere durch jeweils ein Okular für das rechte Auge und das linke Auge, betrachtet. Der Betrachter kann so die beiden Bilder durch die beiden Okulare stereoskopisch, das heisst als dreidimensionales Bild, betrachten.

Durch das Vorsehen der zwei Okulare zum stereoskopischen Betrachten der beiden Bilder wird erreicht, dass die Vorrichtung an sich in bekannter und dem Verwender in gewohnter Form vorliegt. Des Weiteren stellt diese Form der stereoskopischen Darstellung von aufgenommenem Bildmaterial eine besonders einfache Variante dar, insbesondere da der Betrachter sich nicht von herkömmlichen Vorrichtungen zur stereoskopischen Betrachtung eines Auges umgewöhnen muss.

Die Okulare können eine Linse oder ein Linsensystem umfassen. Je nach Ausführung der Bildwiedergabeeinheit können die Okulare auch lediglich als Rohrmantel ausgebildet sein, welcher wahlweise ein Sichtglas zum Schutze der Bildwiedergabeeinheit und/oder eine Lupe zur Vergrösserung des durch die Bildwiedergabeeinheit dargestellten Bildes umfasst.

In Varianten kann auch auf die Okulare verzichtet werden, wobei alternative Betrachtungseinheiten verwendet werden. Zum Beispiel kann ein autostereoskopisches Display eingesetzt werden. Weiter ist auch eine Projektion mittels zwei Projektoren, insbesondere mittels Beamer, und dem Einsatz von Polarisationsfilter denkbar. Die Betrachtungseinheit kann auch lediglich eine Bildwiedergabeeinheit aufweisen, auf welche zum Beispiel mittels Anaglyphenverfahren abgebildet und mittels einer entsprechenden Brille das stereoskopische Bild betrachtet wird. Damit kann zwar ein Bildschirm eingespart werden, anderseits verliert man die Farbechtheit. Dem Fachmann sind dazu auch weitere Techniken bekannt.

Die Betrachtungseinheit ist unabhängig von der Optik bewegbar ausgebildet. Bevorzugt sind die Betrachtungseinheit, insbesondere die Okulare und die Bildwiedergabeeinheiten relativ zur Optik respektive zu den Objektiven, um eine vertikale Achse verschwenkbar ausgebildet. Die Bildwiedergabeeinheit kann um dieselbe Achse verschwenkbar gelagert sein wie die Optik. Damit ergeben sich mehrere Benutzungsmöglichkeiten der Vorrichtung.

In einer ersten Variante kann die Betrachtungseinheit bezüglich des zu betrachtenden Auges, zum Beispiel mittels einer Rastvorrichtung, fixiert sein, während die Optik unabhängig von der Betrachtungseinheit verschwenkbar bleibt. Dadurch kann der Betrachter das Auge betrachten und den Betrachtungswinkel zum Auge ändern, ohne dass die Betrachtungseinheit bewegt werden muss. Damit wird dem Betrachter eine ergonomischere Untersuchung ermöglicht.

In einer zweiten Variante kann zum Beispiel die Betrachtungseinheit mit der Optik reversibel gekoppelt sein. Damit können die Bewegungen der Optik und der Bildwiedergabeeinheit, falls durch den Betrachter gewünscht, in gewohnter Weise gekoppelt sein, was zum Beispiel mittels einer bekannten mechanischen Kopplungsvorrichtung, wie zum Beispiel eines Form- und/oder Kraftschlusses zwischen den beiden Schwenkachsen, zwischen der Optik und der Bildwiedergabeeinheit erreicht werden kann.

In einer dritten Variante können sowohl die Betrachtungseinheit wie auch die Optik unabhängig bewegt werden. Dies kann dann von Vorteil sein, wenn zwei Betrachter vorhanden sind (z. B. Ausbildner und Auszubildender) und der Eine dem Anderen die Betrachtungseinheit zuführen möchte. In diesem Fall müssen nämlich die Plätze nicht getauscht werden. Die Optik kann fixiert bleiben, während die Betrachtungseinheit verschwenkt wird.

In einer vierten Variante kann die Betrachtungseinheit, welche im Wesentlichen die Okulareemit den Mikrobildschirmen umfasst, vom Mikroskop respektive der Optik vollständig losgelöst sein. Eine solche Betrachtungseinheit kann zum Beispiel in der Telemedizin oder zu Ausbildungszwecken Verwendung finden. Die Betrachtungseinheit kann eine Schnittstelle umfassen, womit diese direkt mit einer erfindungsgemässen Spaltlampe, indirekt über einen Computer mit einer erfindungsgemässen Spaltlampe oder lediglich mit einem Computer, welcher über entsprechendes Bilddatenmaterial verfügt, verbunden sein können. Die Betrachtungseinheit kann weiter direkt oder indirekt mit dem Internet oder einem sonstigen Netzwerk verbunden sein, so dass Daten, insbesondere Bilddaten vom Netzwerk bezogen werden können. In einer Anwendung kann ein Augenarzt mit einer digitalen Spaltlame einen Patienten untersuchen, während ein lokal entfernter Experte über eine losgelöste Betrachtungseinheit die Untersuchung mitverfolgen oder sogar beeinflussen kann. In einer weiteren Anwendung kann in einem Praktikum für die Ausbildung zur Bedienung einer Spaltlampe jeder Praktikant mit einer losgelösten Betrachtungseinheit ausgestattet sein, welche mit einer digitalen Spaltlampe, welche vom Dozenten bedient wird, verbunden sind. Damit können die Praktikanten online unter praxisnahen Bedingungen eine Untersuchung an einem Auge mitverfolgen. Dem Fachmann ist klar, dass sämtliche Anwendungen auch mit vollständigen digitalen Spaltlampen durchgeführt werden können, dass aber je nach Einsatz die Verwendung von losgelösten Betrachtungseinheiten aus Kosten- und/oder Platzgründen vorteilhaft sein kann, da damit im Wesentlichen auf das teure Mikroskop verzichtet werden kann.

Die Betrachtungseinheit kann auch als 3D-Video-Brille, das heisst eine Brille, welche zwei integrierte Bildschirme umfasst, ausgebildet sein. Diese kann über Kabel oder kabellos mit dem Bildsensor verbunden sein. Die 3D-Video-Brille kann beispielsweise lösbar in der Vorrichtung gehalten sein, so dass der Betrachter die 3D-Video-Brille wahlweise aus einer Halterung entnehmen und aufsetzen oder in der Halterung lassen und konventionell das Bildmaterial betrachten kann. Die Vorrichtung kann auch die Verwendung von mehreren 3D-Video-Brillen unterstützen. Damit können zum Beispiel Schulungen durchgeführt werden.

Vorzugsweise umfasst die Vorrichtung eine Datenverarbeitungseinheit. Die Datenverarbeitungseinheit umfasst bevorzugt einen Prozessor zum Verarbeiten von Daten, insbesondere der elektronisch erfassten Bilder und einen Speicher zum Speichern der Daten, insbesondere der elektronisch erfassten und/oder der bearbeiteten Bilder. Damit wird erreicht, dass die durch den Bildsensor erfassten Bilder vor oder während der Darstellung zur besseren pathologischen Untersuchung durch die Bildwiedergabeeinheit elektronisch verarbeitet werden können. Vorzugsweise umfassen die Verarbeitung der Daten, insbesondere der elektronisch erfassten Bilddaten, bekannte Bild- und/oder Filmbearbeitungstechniken. Die elektronische Verarbeitung kann dabei eine Bildoptimierung umfassen, wobei zum Beispiel die Kontraste verbessert, Farben geändert, die Helligkeit beeinflusst, das Bild abstrahiert (zum Beispiel Wiedergabe von Höhenkurven) etc. werden können. Weiter kann damit auch ein schematisches dreidimensionales Auge unter Einbeziehen von Daten anderer Messmethoden berechnet werden. Schliesslich kann auch eine residuale Abbildung zu einem idealen Auge errechnet und dargestellt oder ein operativer Eingriff simuliert werden, Dem Fachmann ist klar, dass mittels einer Datenverarbeitung die Bilddaten in nahezu unbeschränkter Weise verarbeitet werden können, insbesondere dass die obige Auflistung nicht abschliessend ist.

Weiter besteht die Möglichkeit, dass die Bilddaten eines Patienten auch in seiner Abwesenheit vom Speicher abgerufen und betrachtet werden können. Dies kann zum Beispiel dann von Vorteil sein, wenn der Untersuchende eine Zweitmeinung in Abwesenheit des Patienten einholen möchte. Mittels des Speichers können die Rohdaten und/oder die verarbeiteten Bilddaten gespeichert werden. Der Prozessor kann eine bekannte Bauform aufweisen und von diversen Herstellern bezogen werden. Natürlich muss es sich bei den Daten nicht ausschliesslich um Bilddaten handeln.

In Varianten kann die Datenverarbeitungseinheit auch einfacher aufgebaut sein, so dass Bildverarbeitungen nicht oder nur beschränkt möglich sind. Dies kann insbesondere dann ausreichend sein, wenn die Vorrichtung eine Schnittstelle zu einer externen Datenverarbeitungsvorrichtung (Computer, Laptop und dergleichen) aufweist, womit die Daten aufbereitet und gespeichert werden können.

Vorzugsweise umfasst die Vorrichtung eine Schnittstelle zum Übertragen von Daten an ein und zum Empfangen von Daten von einem elektronischen Gerät. Damit besteht die Möglichkeit, die Bilddaten an eine Drittperson (zum Beispiel einen Arzt) zu senden oder einem Patienten auf einem Datenträger oder als Ausdruck auszuhändigen. Dadurch, dass Daten auch empfangen werden können wird erreicht, dass Bilddaten respektive auch weitere Daten, wie Einstellungen der Vorrichtung, einer Untersuchung empfangen und auf der eigenen Vorrichtung betrachtet werden können. Bei einer Echtzeitübertragung kann damit bei einer Vorrichtung ein Auge eines Patienten untersucht werden, wobei weitere Personen diese Untersuchung in Echtzeit mitverfolgen können. Dies kann insbesondere bei Schulungen von Interesse sein. Die Schnittstelle kann dabei als Datenleitung, als drahtlose Verbindung (Funk, Infrarot, Bluetooth, etc.) oder weitere dem Fachmann bekannte Schnittstelle ausgebildet sein. Insbesondere können auch mehrere, insbesondere verschiedene Schnittstellen vorgesehen sein.

Alternativ kann auf diese Schnittstelle auch verzichtet werden, insbesondere wenn die Vorrichtung selbst zum Beispiel einen Computer und/oder ein auswechselbares Speichermedium (Memory Stick, Diskette, Harddisk etc.) umfasst.

Vorzugsweise ist das elektronische Gerät als eines oder mehrere der nachfolgenden Geräte ausgebildet:
- eine zweite Vorrichtung zur stereoskopischen Untersuchung eines Auges, insbesondere eine Spaltlampe;
- ein Computer oder ein Laptop, insbesondere ein Computernetzwerk;
- ein Bildschirm, insbesondere ein Bildschirm zur stereoskopischen Betrachtung der Bilder, vorzugsweise ein autostereoskopisches Display zu stereoskopischen Wiedergabe des Bildmaterials;
- ein PDA (personal digital assistant), iPad oder ähnliches;
- Massenspeicher, zum Beispiel eine externe Harddisk.

An die Vorrichtung kann direkt oder indirekt, zum Beispiel über einen Computer, ein oder mehrere Projektoren (jeweils für ein Halbbild) oder ein Display, insbesondere ein herkömmlicher Bildschirm, Fernseher oder Flachbildschirm angeschlossen sein, womit die Bilddaten dargestellt werden können. Die Bilddaten können mittels eines Bildschirms zweidimensional dargestellt werden, indem nur eines der beiden Bilder dargestellt wird. Alternativ können die Bilddaten der beiden Bilder zu einem Anaglyphenbild, insbesondere in ein Farbanaglyphenbild, umgewandelt und am Bildschirm dargestellt werden, so dass das Bildmaterial mit einer geeigneten Brille als dreidimensionale Darstellung betrachtet werden kann. Ein autostereoskopisches Display kann dazu natürlich auch verwendet werden. Weiter kann auch eine Shutterbrille verwendet werden, wobei der Bildschirm in hoher Frequenz alternierend die Halbbilder darstellt und synchron dazu die Shutterbrille jeweils ein Auge abdeckt. Eine Projektion der beiden Halbbilder durch Polarisationsfilter auf eine, bei einer Lichtreflektion die Polarisationsebene erhaltende Oberfläche, wobei der Betrachter eine entsprechende Polarisationsbrille aufsetzt, ist auch denkbar. Weiter kann die Vorrichtung direkt oder indirekt mit einer 3D-Video-Brille über Kabel oder kabellos verbunden sein, wobei die 3D-Video-Brille selbst zwei Mikrobildschirme umfasst. Dem Fachmann sind weitere Darstellungsmöglichkeiten für dreidimensionale Bilder bekannt. Die Bilddaten können sowohl in Echtzeit oder nachträglich über ein Display oder einen Projektor betrachtet werden.

Falls die Vorrichtung über einen Datenkanal mit einem Computer verbunden ist, können die Daten natürlich direkt vom Computer auf weitere bekannte elektronische Vorrichtungen, wie zum Beispiel einen PDA, einen Memory Stick, eine CD, eine DVD, eine Blueray, eine Harddisk, insbesondere eine externe Harddisk etc. übertragen werden. Bei einer weiteren vorteilhaften Ausgestaltung der Vorrichtung umfasst jedoch die Vorrichtung selbst eine Schnittstelle, mittels welcher eine oder mehrere der obig genannten elektronischen Vorrichtungen oder weitere dem Fachmann bekannte Vorrichtungen direkt zum Datenaustausch verbunden werden können. Als Beispiel sei hier ein USB-Anschluss eines beliebigen Typs erwähnt. In der Bildwiedergabeeinheit kann zum Beispiel ein Betriebssystem aufrufbar sein, womit die Daten verwaltet werden können. Ein Steuergerät der Vorrichtung, zum Beispiel der Joystick zur Bedienung des Kreuzschlittens kann dazu in einer separaten Einstellung die Funktion der Maus übernehmen, so dass ein Cursor auf den Mikrodisplays der Bildwiedergabeeinheit geführt werden kann. Die Daten können auch automatisch auf einem herkömmlichen Speichermedium gespeichert werden.

Dem Fachmann sind auch weitere Geräte bekannt, welche über einen Datenkanal mit der Vorrichtung verbunden werden können. Diese umfassen auch weitere Untersuchungsgeräte, wie zum Beispiel ein Tonometer oder dergleichen. Damit können die durch die verschiedenen Messmethoden ermittelten Daten kombiniert werden.

Vorzugsweise können über die Schnittstelle elektronisch erfasste Bilder der zweiten Vorrichtung zur stereoskopischen Untersuchung eines Auges empfangen werden und sind mittels der Bildwiedergabeeinheit wiedergebbar. Vorzugsweise ist die zweite Vorrichtung zur stereoskopischen Untersuchung eines Auges ebenfalls mit einer Bildwiedergabeeinheit mit elektronischen Bildschirmen ausgestattet. Damit kann, zum Beispiel für Schulungszwecke, das Bildmaterial zwischen den Vorrichtungen versandt und mittels der Vorrichtung betrachtet werden. Weiter können auch Einstellungsdaten der Vorrichtung (Lampeneinstellungen, Spaltdimensionen etc.) versandt werden, welche entweder automatisch oder manuell eingestellt werden können. Auch eine Identifizierung der Vorrichtungen (Daten wurden von Spaltlampe XY gesandt) kann über die versendbaren Daten erreicht werden.

Die Daten werden nicht unbedingt direkt an eine Vorrichtung zur stereoskopischen Untersuchung eines Auges gesandt, sondern können auch via einen Computer oder ein Computernetzwerk an eine solche Vorrichtung versandt werden, wobei die Daten wahlweise mittels Computer vorgängig bearbeitet werden können. Insbesondere können die Vorrichtungen über ein herkömmliches Netzwerk verbunden sein. Natürlich muss am Computer oder am Computernetzwerk nicht zwingend eine weitere solche Vorrichtung angeschlossen sein.

Die Daten umfassen vorzugsweise elektronisch erfasste Bilder, Uhrzeit, Datum und/oder Einstellungsdaten der Vorrichtung. Die Einstellungsdaten umfassen vorzugsweise weiter die Spaltbreite, die Spalthöhe, eingesetzte Filtertypen, die Belichtungsmethode und/oder den Vergrösserungsfaktor. Datum und Uhrzeit können beispielsweise automatisch mit den Bilddaten verknüpft werden, um das Auffinden zu vereinfachen.

Dem Fachmann ist klar, dass nicht sämtliche obig aufgezählten Datentypen zur Übertragung vorgesehen sein müssen.

Bevorzugt sind die Bilder wahlweise statisch als Bild, dynamisch als Film oder dynamisch als Film in Echtzeit über die Betrachtungseinheit wiedergebbar. Die Vorrichtung kann so beschaffen sein, dass während des Betrachtens ein Bild auch zur statischen Betrachtung "eingefroren" werden kann. Gespeichertes Bildmaterial kann auch im Nachhinein zum Beispiel mittels einer entsprechend ausgerüsteten Spaltlampe abgerufen und statisch als Bilder oder dynamisch als Filmaufnahme betrachtet werden.

In einem zweiten Aspekt der Erfindung umfasst eine Vorrichtung zur Untersuchung eines Auges, insbesondere ein Spaltlampenmikroskop, vorzugsweise eine Optik zur Erzeugung eines Bildes, einen Bildsensor zur elektronischen Erfassung eines Bildes und eine Betrachtungseinheit mit einer Bildwiedergabeeinheit zur Darstellung des Bildes sowie ein Okular zur Betrachtung des Bildes.

Dem Fachmann ist hierbei klar, dass die obig aufgeführten bevorzugten Merkmale ebenso mit dem zweiten Aspekt der Erfindung kombinierbar sind, ausser denjenigen Merkmalen, welche sich unbedingt auf die stereoskopische Anwendung beziehen.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine schematische Darstellung einer Seitenansicht einer ersten Ausführungsform einer Vorrichtung zur Untersuchung eines Auges;
- Fig. 2: eine vereinfachte Schnittdarstellung durch die Optik und die Bildwiedergabeeinheit einer erfindungsgemässen Vorrichtung zur stereoskopischen Untersuchung eines Auges;
- Fig. 3: eine zweite Ausführungsform einer erfindungsgemässen Vorrichtung gemäss einem ersten Aspekt der Erfindung;
- Fig. 4: eine dritte Ausführungsform der erfindungsgemässen Vorrichtung in einer Seitenansicht;
- Fig. 5: eine Betrachtungseinheit als vom Mikroskop losgelöstes Element.

Grundsätzlich sind in den Figuren gleiche Teile mit gleichen Bezugszeichen versehen.

### Wege zur Ausführung der Erfindung

Der nachfolgend verwendete Begriff Lichtstrahl ist nicht auf einen einzigen Lichtstrahl respektive auf ein Photon eingeschränkt zu verstehen, sondern auch als ein Lichtweiienbündei verstanden, welches ein Bild repräsentieren kann.

Die Figur 1 zeigt eine Vorrichtung 1 zur Untersuchung eines Auges, ausgebildet als Spaltlampe 1. Die Spaltlampe 1 umfasst eine Beleuchtungseinheit 100, einen Optikteil 220, eine Betrachtungseinheit 200 welche auf einem Kreuzschlitten 300 montiert sind.

Der Kreuzschlitten 300 selbst ist auf einer Grundplatte 400 montiert, welche als Tischplatte ausgebildet sein kann, und kann in X-, Y- und Z-Richtung verfahren werden. Der Schlitten 300 ist über ein Bedienelement 401, welches an demselben angeordnet ist, steuerbar. Die Beleuchtungseinheit 100 und die Betrachtungseinheit 200 sind damit über den Schlitten 300 verfahrbar sowie über eine gemeinsame Drehachse 600 unabhängig voneinander verschwenkbar angeordnet.

Die Beleuchtungseinheit 100 umfasst ein L-förmiges Element 110 mit einem horizontalen Abschnitt 111 und einem vertikalen Abschnitt 112. In einem distalen Bereich des horizontalen Abschnittes 111 umfasst das L-förmige Element 110 eine vertikal orientierte Drehachse 600. Oben auf dem vertikalen Abschnitt ist eine Beleuchtungsvorrichtung 120, umfassend eine Lichtquelle 121, angeordnet. Die Beleuchtungseinheit 100 ist so beschaffen, dass ein definierter Lichtstreifen erzeugbar ist, welcher auf ein Auge 810 projiziert werden kann. An der Innenseite des L-förmigen Elements 110, am vertikalen Abschnitt 112 befindet sich ein Spiegel 130, welcher sich vom vertikalen Abschnitt 112 aus in einem Winkel von 45° neigt. Ein durch die Lichtquelle 121 erzeugter Lichtstrahl 140 wird senkrecht nach unten auf den in einem 45°-Winkel angeordneten Spiegel 130 geleitet und von diesem zum Auge 810 des Patienten 800 geleitet.

Die in der Figur 1 gezeigte Ausführungsform umfasst ein L-förmiges Element 210 mit einem horizontalen Abschnitt 211 und einem vertikalen Abschnitt 212. In einem distalen Bereich des horizontalen Abschnittes 211 und unterhalb des horizontalen Abschnittes 111 des L-förmigen Elements 110 ist das L-förmige Element 210 auf der Drehachse 600 gelagert. Die beiden L-förmigen Elementen 110, 210 sind damit gewissermassen aufeinander gestapelt und weisen eine gemeinsame Drehachse 600 auf. Oben, auf dem vertikalen Abschnitt 212 ist ein Optikteil 220 angeordnet, in welche die vom Auge 810 reflektierten Lichtstrahlen 140 gelangen. Der Optikteil 220 ist im Wesentlichen als Biomikroskop ausgebildet, welches zwei Halbbilder eines stereoskopischen Bildes aufnehmen kann. Der Optikteil 220 umfasst in der vorliegenden Ausführungsform aber zwei halbdurchlässige Spiegel 230, welcher in Richtung der Lichtstrahlen 140, welche durch das Auge 810 reflektiert werden, in einem Winkel von 45° nach unten geneigt sind. Mittels dieser halbdurchlässigen Spiegel 230 werden die vom Auge 810 reflektierten Lichtstrahlen jeweils in zwei Teilstrahlen aufgeteilt.

Jeweils ein Teilstrahl wird nach unten in eine Bildaufnahmeeinheit 240 geleitet. Die Bildaufnahmeeinheit 240 umfasst zwei Bildsensoren 241 zur elektronischen Erfassung der durch die beiden Teilstrahlen repräsentierten Bilder. Die Bildaufnahmeeinheit 240 kann auch weitere Komponenten, wie zum Beispiel ein Linsensystem, eine Blende etc. umfassen. Die Bildsensoren 241 sind über einen Datenkanal mit einem Bildschirm 700 verbunden. Mittels des Bildschirms 700 können die Bilddaten der Bildsensoren 241 mit bekannten Techniken (Anaglyphenverfahren, autostereoskopisches Display etc.) dreidimensional dargestellt werden. Natürlich können sie auch zweidimensional dargestellt werden. Für die Aufarbeitung der Bilddaten ist eine Datenverarbeitungsvorrichtung vorgesehen (hier nicht dargestellt).

Die beiden weiteren Teilstrahlen werden über zwei Umlenkspiegel 231, welche auch als Prisma realisiert werden können, jeweils in ein Okular 260 einer Bildwiedergabeeinheit 250 geleitet, wodurch die Bilder durch einen Untersuchenden 900 auch analog, insbesondere in der "altbekannten" Form betrachtet werden können.

Auf detaillierte Darstellungen der optischen Bauteile, insbesondere von Linsensystemen zur Vergrösserung und Fokusierung, Filter etc wird bewusst verzichtet, da dem Fachmann deren Aufbau hinreichend bekannt ist.

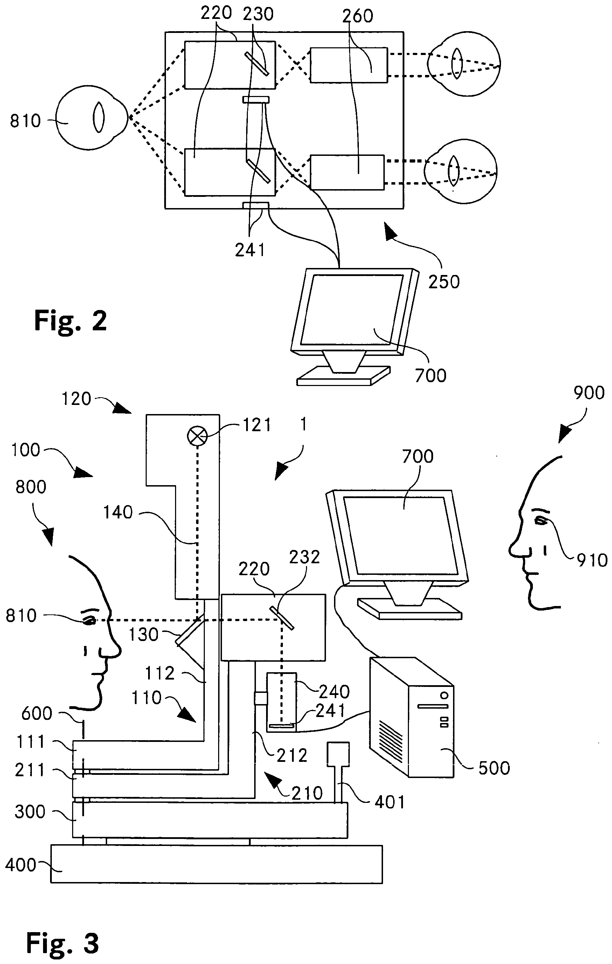

Die Figur 2 zeigt eine vereinfachte schematische Darstellung des Strahlengangs gemäss Figur 1. Die vom Auge 810 reflektierten Lichtstrahlen, welche jeweils ein linkes und ein rechtes Halbbild umfassen, welche in Kombination eine stereoskopische Darstellung ermöglichen, gelangen jeweils in einen Optikteil 220, welcher jeweils ein nicht dargestelltes Linsensystem umfassen kann, zum halbdurchlässigen Spiegel 230. Von diesen werden die Lichtstrahlen, wie obig beschrieben, jeweils in zwei Teilstrahlen aufgeteilt, wobei jeweils ein Teilstrahl zu den Bildsensoren 241 und der zweite Teilstrahl jeweils durch den halbdurchlässigen Spiegel 230 hindurch in die Okulare 260 der Betrachtungseinheit 250 geleitet wird. Mittels der Okulare 260 kann der Untersuchende 900 die Bilder direkt und analog stereoskopisch betrachten. Die Bildsensoren 241 sind über Datenkanäle mit einem Bildschirm 700 verbunden, womit ein Untersuchender 900 das Bildmaterial in digital verarbeiteter Form oder als Rohdaten betrachten kann. In Varianten können die Okulare auch durch Bildsensoren ersetzt werden, womit auf die halbdurchlässigen Spiegel 230 verzichtet werden kann. Weiter können die Okulare 260 mit Mikrobildschirmen ausgestattet sein, welche mit den Bildsensoren 241 verbunden sind, wobei damit statt der halbdurchlässigen Spiegel 230 undurchlässige respektive vollständig reflektierende Spiegel eingesetzt werden (siehe unten).

Die Figur 3 zeigt im Wesentlichen die Vorrichtung gemäss Figur 1, wobei, wie oben erwähnt, die halbdurchlässigen Spiegel 230 der Figur 2 durch Spiegel 232 ersetzt sind, welche nicht durchlässig ist, womit auch die Okulare 260 obsolet und damit in dieser Ausführungsform nicht vorgesehen sind. Die Bilddaten, welche die beiden Halbbilder repräsentieren, werden von den beiden Bildsensoren 241 in der vorliegenden Ausführungsform an einen Computer 500 gesandt. In der Figur 2 ist dies mit einem Kabel dargestellt, wobei aber auch eine drahtlose Übertragung denkbar ist. Mittels des Computers 500 können die Bilddaten gespeichert, bearbeitet und zum Beispiel über ein Netzwerk (nicht dargestellt) weiteren Verwendern zur Verfügung gestellt werden. Der Computer 500 ist wiederum mit einem Bildschirm 250 verbunden, womit die Bilddaten entweder zwei- oder dreidimensional dargestellt werden können.

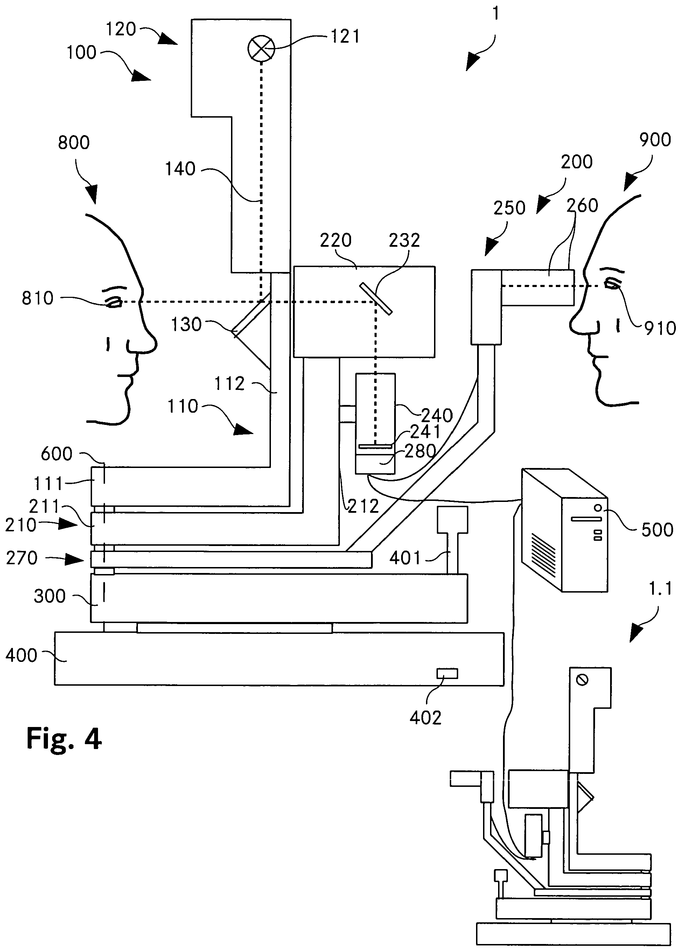

Die Figur 4 entspricht im Wesentlichen der Figur 3, wobei in der vorliegenden Ausführungsform zusätzlich ein weiteres im Wesentlichen L-förmiges Element 270, ähnlich den L-förmigen Elementen 110, 210, zwischen dem Schlitten 300 und dem L-förmiges Element 210 an der Drehachse 600 verschwenkbar gelagert ist. In einem oberen Bereich des L-förmigen Elements 270 ist eine Bildwiedergabeeinheit 250 mit zwei Okulare 260 angeordnet. Die Okulare 260 umfassen jeweils einen Mikrobildschirm. Die Mikrobildschirme sind jeweils über einen Datenkanal, in der vorliegenden Ausführungsform über Kabel, wobei aber auch eine drahtlose Übertragung möglich ist, mit den Bildsensoren 241 der Bildaufnahmeeinheit 240 verbunden. Die Okulare 260, zusammen mit den Mikrobildschirmen können auch als eine Brille, zum Beispiel als eine 3D-Videobrille, ausgebildet sein, welche mit dem L-förmigen Element 270 lösbar verbunden werden kann. Die Vorrichtung kann weiter selbst eine Datenverarbeitungseinheit 280 umfassen, womit die Bilddaten verarbeitet werden können. Die Datenverarbeitungseinheit 280 kann so beschaffen sein, dass ein Betriebssystem ausführbar ist, welches über die Mikrobildschirme der Okulare 260 und/oder über einen externen zusätzlichen Bildschirm 700 und ein Joystick, insbesondere das Bedienelement 401 gesteuert werden kann. Über eine Schnittstelle 402 können die Daten an weitere Geräte, insbesondere an eine 3D-Videobrille, einen Computer, eine digitale Spaltlampe 1 etc. gesandt werden. In der Figur 4 ist als Peripheriegerät exemplarisch eine Spaltlampe 1.1 dargestellt, welche über einen Computer 500 mit der Vorrichtung 1 verbunden ist.

Gemäss eines zweiten Aspekts der Erfindung kann die Vorrichtung 1 gemäss Figur 4 auch lediglich zur monoskopischen Betrachtung von Bildern vorgesehen sein. Dazu umfasst die Vorrichtung 1 einen Optikteil 220 zur Aufnahme genau eines Bildes, sowie genau einen Bildsensor 241 zur Erfassung eines Bildes und die Betrachtungseinheit kann dabei ein oder zwei Okulare 260 umfassen, mit welchem/mit welchen das eine Bild auf dem Bildschirm betrachtet werden kann.

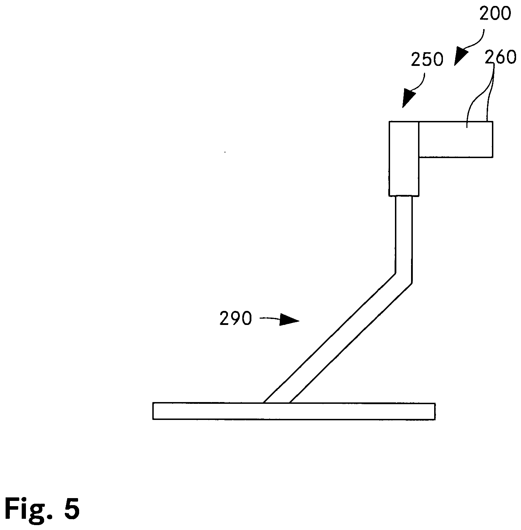

Schliesslich zeigt die Figur 5 eine Betrachtungseinheit 200 als von dem Mikroskop losgelöste Einheit. Die Betrachtungseinheit 200 ist in der vorliegenden Ausführungsform auf einem Ständer 290 montiert, welche in nicht dargestellter Weise in der Höhe verstellbar ist, so dass die Arbeitshöhe dem Benutzer angepasst werden kann. Es versteht sich dabei, dass der Ständer 290 der Betrachtungseinheit 200 nicht auf die schematische Darstellung in der Figur 5 beschränkt ist. Dem Fachmann ist klar, dass ein solcher Ständer 290 beliebig ausgebildet sein kann. Die Betrachtungseinheit kann über einen Datenkabel oder drahtlos mit Bilddaten versorgt werden. Weiter kann die Betrachtungseinheit eine elektronische Steuerung und eine Eingabeeinheit umfassen (nicht dargestellt), womit die Mikrobildschirme bedient und gesteuert werden können. Damit können beispielsweise die Helligkeit, der Kontrast und weitere dem Fachmann bekannte Einstellungen der Mirkobildschirme vorgenommen werden. Weiter können auch ein Prozessor und ein Speicher vorgesehen sein, womit das Bildmaterial über die Bedienelemente bearbeitet und gespeichert werden kann. Mehrere solcher Betrachtungseinheiten können zusammen so gekoppelt sein, so dass Daten untereinander ausgetauscht werden können. Diese Ausführungsform kann insbesondere in der Telemedizin und für Schulungszwecke vorteilhaft sein.

Dem Fachmann ist klar, dass in den vorliegenden Ausführungsbeispielen zum Beispiel auch genau ein Bildsensor vorgesehen sein kann.

Zusammenfassend ist festzustellen, dass erfindungsgemäss eine Vorrichtung zur stereoskopischen respektive zur monoskopischen Untersuchung eines Auges geschaffen wird, womit Bildmaterial aufgenommen, bearbeitet und wieder stereoskopisch respektive monoskopisch betrachtet werden kann.

## Patentansprüche

1. Spaltlampenmikroskop (1) zur stereoskopischen Untersuchung eines Auges (810) mit einer Optik (220) zur Erzeugung zweier Bilder des Auges (810), wobei das Spaltlampenmikroskop (1) wenigstens einen, vorzugsweise zwei Bildsensoren (241) zur elektronischen Erfassung der beiden Bilder umfasst, wobei sie weiter eine Betrachtungseinheit (200) mit einer Bildwiedergabeeinheit (250) zur Darstellung der beiden Bilder sowie zwei Okulare (260) zur stereoskopischen Betrachtung der Bilder umfasst, **dadurch gekennzeichnet, dass** die Betrachtungseinheit (200) unabhängig von der Optik (220) bewegbar ausgebildet ist.

2. Spaltlampenmikroskop (1) nach Anspruch 1, **dadurch gekennzeichnet, dass** sie eine Datenverarbeitungseinheit (280) umfasst.

3. Spaltlampenmikroskop (1) nach Anspruch 2, **dadurch gekennzeichnet, dass** die Datenverarbeitungseinheit (280) einen Prozessor zum Verarbeiten von Daten, insbesondere der elektronisch erfassten Bilder und einen Speicher zum Speichern der Daten, insbesondere der elektronisch erfassten und/oder der bearbeiteten Bilder umfasst.

4. Spaltlampenmikroskop (1) nach einem der Ansprüche 1 bis 3, **dadurch gekennzeichnet, dass** sie eine Schnittstelle (402) zum Übertragen von Daten an ein und zum Empfangen von Daten von einem elektronischen Gerät umfasst.

5. Spaltlampenmikroskop (1) nach Anspruch 4, **dadurch gekennzeichnet, dass** das elektronische Gerät als eines oder mehrere der nachfolgenden Geräte ausgebildet ist:
a) ein zweites Spaltlampenmikroskop (1) zur stereoskopischen Untersuchung eines Auges (810), insbesondere ein Spaltlampenmikroskop (1) nach einem der Ansprüche 1 bis 4;
b) ein Computer (500), insbesondere ein Computernetzwerk;
c) ein Bildschirm (700), insbesondere ein Bildschirm zur stereoskopischen Betrachtung der Bilder, vorzugsweise ein autostereoskopisches Display zu stereoskopischen Wiedergabe der beiden Bilder.

6. Spaltlampenmikroskop (1) nach Anspruch 5, **dadurch gekennzeichnet, dass** über die Schnittstelle (402) elektronisch erfasste Bilder des zweiten Spaltlampenmikroskops (1) zur stereoskopischen Untersuchung eines Auges (810) empfangen werden können und mittels der Bildwiedergabeeinheit (250) wiedergebbar sind.

7. Spaltlampenmikroskop (1) nach einem der Ansprüche 2 bis 6, **dadurch gekennzeichnet, dass** die Daten elektronisch erfasste Bilder, Uhrzeit, Datum und/oder Einstellungsdaten des Spaltlampenmikroskops (1) umfassen.

8. Spaltlampenmikroskop (1) nach Anspruch 7, **dadurch gekennzeichnet, dass** die Daten Folgendes umfassen:
a) eine Spaltbreite;
b) eine Spalthöhe;
c) eingesetzte Filtertypen;
d) eine Belichtungsmethode; und/oder
e) einen Vergrösserungsfaktor.

9. Spaltlampenmikroskop (1) nach einem der Ansprüche 1 bis 8, **dadurch gekennzeichnet, dass** die Bilder wahlweise statisch als Bild, dynamisch als Film oder dynamisch als Film in Echtzeit über die Betrachtungseinheit (200) wiedergebbar sind.

10. Spaltlampenmikroskop (1) zur Untersuchung eines Auges (810) mit einer Optik (220) zur Erzeugung eines Bildes, wobei sie einen Bildsensor (241) zur elektronischen Erfassung eines Bildes umfasst, wobei sie eine Betrachtungseinheit (200) mit einer Bildwiedergabeeinheit (250) zur Darstellung des Bildes sowie ein Okular (260) zur Betrachtung des Bildes umfasst, **dadurch gekennzeichnet, dass** die Betrachtungseinheit (200) unabhängig von der Optik (220) bewegbar ausgebildet ist.

## Claims

1. A slit-lamp microscope (1) for stereoscopically examining an eye (810), having an optical unit (220) for generating two images of the eye (810), wherein the slit-lamp microscope (1) comprises at least one, preferably two image sensors (241) for electronic capture of the two images, wherein it comprises an observation unit (200) with an image rendering unit (250) for displaying the two images and two eyepieces (260) for stereoscopically observing the images, **characterized in that** the observation unit (200) is embodied to be movable independently of the optical unit (220).

2. The slit-lamp microscope (1) as claimed in claim 1, **characterized in that** it comprises a data processing unit (280).

3. The slit-lamp microscope (1) as claimed in claim 2, **characterized in that** the data processing unit (280) comprises a processor for processing data, in particular the electronically captured images, and a storage device for storing the data, in particular the electronically captured and/or the processed images.

4. The slit-lamp microscope (1) as claimed in one of claims 1 to 3, **characterized in that** it comprises an interface (402) for transmitting data to an electronic instrument and for receiving data therefrom.

5. The slit-lamp microscope (1) as claimed in claim 4, **characterized in that** the electronic instrument is embodied as one or more of the following instruments:
a) a second slit-lamp microscope (1) for stereoscopically examining an eye (810), in particular a slit-lamp microscope (1) as claimed in one of claims 1 to 4;
b) a computer (500), in particular a computer network;
c) a screen (700), in particular a screen for stereoscopically observing the images, preferably an autostereoscopic display for stereoscopic rendering of the two images.

6. The slit-lamp microscope (1) as claimed in claim 5, **characterized in that** the interface (402) can be used to receive electronically captured images from the second slit-lamp microscope (1) for the purpose of stereoscopically examining an eye (810), and these images can be rendered by means of the image rendering unit (250).

7. The slit-lamp microscope (1) as claimed in one of claims 2 to 6, **characterized in that** the data comprise electronically captured images, time, date and/or setting data of the slit-lamp microscope (1).

8. The slit-lamp microscope (1) as claimed in claim 7, **characterized in that** the data comprise the following:
a) a slit width;
b) a slit height;
c) utilized filter types;
d) an exposure method; and/or
e) a magnification factor.

9. The slit-lamp microscope (1) as claimed in one of claims 1 to 8, **characterized in that** the images can, by means of the observation unit (200), be selectively rendered statically as an image, dynamically as a film or dynamically as a film in real time.

10. A slit-lamp microscope (1) for examining an eye (810), having an optical unit (220) for generating an image, wherein it comprises an image sensor (241) for electronically capturing an image, wherein is comprises an observation unit (200) with an image rendering unit (250) for displaying the image, and also an eyepiece (260) for observing the image, **characterized in that** the observation unit (200) is embodied to be movable independently of the optical unit (220).

## Revendications

1. Microscope à lampe à fente (1) destiné à l'examen stéréoscopique d'un oeil (810), comprenant une optique (220) servant à générer deux images de l'oeil (810), le microscope à lampe à fente (1) comprenant au moins un, de préférence deux capteurs d'image (241) servant à la capture électronique des deux images, celui-ci comprenant en outre une unité d'observation (200) munie d'une unité de restitution d'image (250) destinée à représenter les deux images ainsi que de deux oculaires (260) destinés à l'observation stéréoscopique des images, **caractérisé en ce que** l'unité d'observation (200) est configurée pour pouvoir être déplacée indépendamment de l'optique (220).

2. Microscope à lampe à fente (1) selon la revendication 1, **caractérisé en ce qu'**il comprend une unité de traitement de données (280).

3. Microscope à lampe à fente (1) selon la revendication 2, **caractérisé en ce que** l'unité de traitement de données (280) comprend un processeur destiné au traitement des données, notamment des images capturées électroniquement, et une mémoire destinée à mémoriser les données, notamment les images capturées électroniquement et/ou traitées.

4. Microscope à lampe à fente (1) selon l'une des revendications 1 à 3, **caractérisé en ce qu'**il comprend une interface (402) destinée à la transmission de données à un appareil électronique et à la réception de données de la part de celui-ci.

5. Microscope à lampe à fente (1) selon la revendication 4, **caractérisé en ce que** l'appareil électronique est réalisé sous la forme d'un ou plusieurs des appareils suivants :
a) un deuxième microscope à lampe à fente (1) destiné à l'examen stéréoscopique d'un oeil (810), notamment un microscope à lampe à fente (1) selon l'une des revendications 1 à 4 ;
b) un ordinateur (500), notamment un réseau d'ordinateurs ;
c) un écran (700), notamment un écran destiné à la représentation stéréoscopique des images, de préférence un afficheur autostéréoscopique destiné à la restitution stéréoscopique des deux images.

6. Microscope à lampe à fente (1) selon la revendication 5, **caractérisé en ce que** les images du deuxième microscope à lampe à fente (1) destiné à l'examen stéréoscopique d'un oeil (810) capturées électroniquement peuvent être reçues par le biais de l'interface (402) et peuvent être restituées au moyen de l'unité de restitution d'image (250).

7. Microscope à lampe à fente (1) selon l'une des revendications 2 à 6, **caractérisé en ce que** les données comprennent des images capturées électroniquement, l'heure, la date et/ou des données de réglage du microscope à lampe à fente (1).

8. Microscope à lampe à fente (1) selon la revendication 7, **caractérisé en ce que** les données comprennent ce qui suit :
a) une largeur de fente ;
b) une hauteur de fente ;
c) les types de filtre utilisés ;
d) une méthode d'éclairage ; et/ou
e) un facteur de grossissement.

9. Microscope à lampe à fente (1) selon l'une des revendications 1 à 8, **caractérisé en ce que** les images peuvent être restituées, au choix, de manière statique sous forme d'image, de manière dynamique sous forme de film ou de manière dynamique sous forme de film en temps réel par le biais de l'unité d'observation (200).

10. Microscope à lampe à fente (1) destiné à l'examen stéréoscopique d'un oeil (810), comprenant une optique (220) servant à générer une image, celle-ci comprenant un capteur d'image (241) servant à la capture électronique d'une image, celle-ci comprenant une unité d'observation (200) munie d'une unité de restitution d'image (250) destinée à représenter l'image ainsi qu'un oculaire (260) destiné à l'observation de l'image, **caractérisé en ce que** l'unité d'observation (200) est configurée pour pouvoir être déplacée indépendamment de l'optique (220).
